# EUROPEAN PATENT APPLICATION

(11) **EP 4 454 470 A1**
(43) Date of publication of application: **30.10.2024**
(21) Application number: 24172815.3
(22) Date of filing: 26.04.2024
(51) Int. Cl.: A23B 7/152

(54) **A METHOD FOR PROVIDING AN ETHYLENE ENVIRONMENT IN A CROP STORE AND AN ETHYLENE ENVIRONMENT CONTROL SYSTEM**

(30) Priority: 26.04.2023 GB 202306173
(71) Applicant: Restrain Company Limited, March, Cambridgeshire PE15 0UW (GB)
(72) Inventor: Briddon, Adrian, Spalding, PE11 2AB (GB); Platteel, Martijn, 3544NH Utrecht (NL)
(74) Representative: Williams Powell

(57) **Abstract**

A method for providing an ethylene environment in a crop store and an ethylene environment control system are disclosed. The method includes monitoring ethylene concentration in the crop store, setting an ethylene acclimatisation dosage to an initial value, performing pump control including repeating applying an ethylene dosage to the crop store according to the acclimatisation dosage; waiting for a predetermined period; and, increasing the acclimatisation dosage value for a next dosage. Upon the monitoring detecting an ethylene concentration for a qualification period the method includes changing from pump control to sensor control, sensor control comprising monitoring ethylene concentration in the crop store and upon the ethylene concentration falling below a predetermined threshold, triggering addition of ethylene to the crop store.

## Description

### Field of the Invention

The present invention relates to an improvement in the method for introducing ethylene(C2H4) in store to a crop (principally potatoes) that reduces the respiration response to this natural plant hormone.

### Background

Ethylene is a natural plant hormone gas that can effectively control sprouting in stored potatoes (and other crops). Commercial systems are available for installation in buildings that allow ethylene to be introduced and the concentration to be managed within pre-determined limits.

Currently the method for introduction of ethylene to commercial potato stores relies on the introduction of a continual supply of C2H4 in the store atmosphere, using a ramped or stepped increase in the level of C2H4. Starting at 0.1ppm, and steadily increasing over 21 days to 2-30ppm, the small step increases reduce the respiration response to C2H4 and conversion of starch to reducing sugars. Increased reducing sugars is commercially undesirable due to the risk of darker fry colour and the increased risk of acrylamide production during high temperature cooking. Increases in respiration also result in an increase in heat and carbon dioxide output of the stored crop. In potato stores it is important to keep temperature and carbon dioxide levels within strict limits and so management interventions are required to maintain temperature and carbon dioxide levels. Failure to maintain appropriate conditions result in crop quality losses.

Management intervention consists of the use of cold ambient air or refrigeration for maintenance of temperature and ambient air for maintenance of carbon dioxide levels. Interventions have a direct cost, the energy cost to operate fans, louvres and refrigeration systems; and an indirect cost, for example the replacement of high humidity store air with low humidity ambient air to reduce carbon dioxide concentration, exacerbates weight loss.

Once stored crops have become accustomed to exposure to ethylene, sensitivity (the increase in respiration rate response) reduces and larger changes in concentration can take place. These changes do not influence the sprout control properties of ethylene.

However it is documented that some potato varieties are more sensitive to ethylene introduction than others and that the current method does not achieve the desired quality aspects. One example of this is the potato cultivar Innovator. This variety is mostly not treated with ethylene due to the quality effects from its respiration response.

### Statement of the Invention

According to an aspect of the present invention, there is provided a method for providing an ethylene environment in a crop store comprising:
monitoring ethylene concentration in the crop store
setting an ethylene acclimatisation dosage to an initial value;
performing pump control including repeating:
   applying an ethylene dosage to the crop store according to the acclimatisation dosage;
   waiting for a predetermined period; and,
   increasing the acclimatisation dosage value for a next dosage;
upon the monitoring detecting an ethylene concentration for a qualification period, changing from pump control to sensor control, sensor control comprising:
   monitoring ethylene concentration in the crop store; and,
   upon the ethylene concentration falling below a predetermined threshold, triggering addition of ethylene to the crop store

During pump control, the applying dosage and waiting for a predetermined period are repeated, with the acclimatisation dosage value being increased for each subsequent dosage step.

According to another aspect of the present invention, there is provided an ethylene environment control system comprising:
a crop store;
an ethylene sensor configured to monitor ethylene concentration in the crop store;
an ethylene source having an ethylene dosage set to an acclimatisation value, the acclimatisation value being below the concentration detectable by the ethylene sensor;
a controller, wherein the controller is configured to:
   monitor the ethylene sensor for detection of ethylene;
   performing pump control including repeating:
      causing the ethylene source to apply an ethylene dosage to the crop store according to the acclimatisation dosage;
      waiting for a predetermined period;
      increasing the acclimatisation dosage value for a next dosage;
upon the ethylene sensor detecting an ethylene concentration for a qualification period, changing from pump control to sensor control, sensor control comprising:
monitoring ethylene concentration in the crop store using the ethylene sensor;
upon the ethylene concentration falling below a predetermined threshold, triggering addition of ethylene to the crop store by the ethylene source.

In embodiments of the present invention, an initial introduction of ethylene is made, whereby crops are exposed to a super low dose (between 0.001-0.099ppm) for a short period between 1-10hr every 24 hours. The store atmosphere returns to approximately 0 (zero) ppm ethylene by natural leakage before the next super low dose in the following 24 hours.

The daily super low dose concentration is increased over a number of days during the pump control period (1), increasing concentrations of ethylene in the store atmosphere in a series of phases with a period of recovery to approximately 0ppm ethylene. Thereafter, a sensor control phase takes effect and under the control of a sensor arrangement, the ethylene application commences a ramped / stepped continual application of ethylene (2)

As a result of the new super low dose phase (1), respiration response by varieties such as cultivar Innovator are reduced and commercial quality goals achieved more easily than compared to the current commercial ethylene method. Furthermore all varieties benefit from this more gentle introduction of ethylene.

### Detailed Description

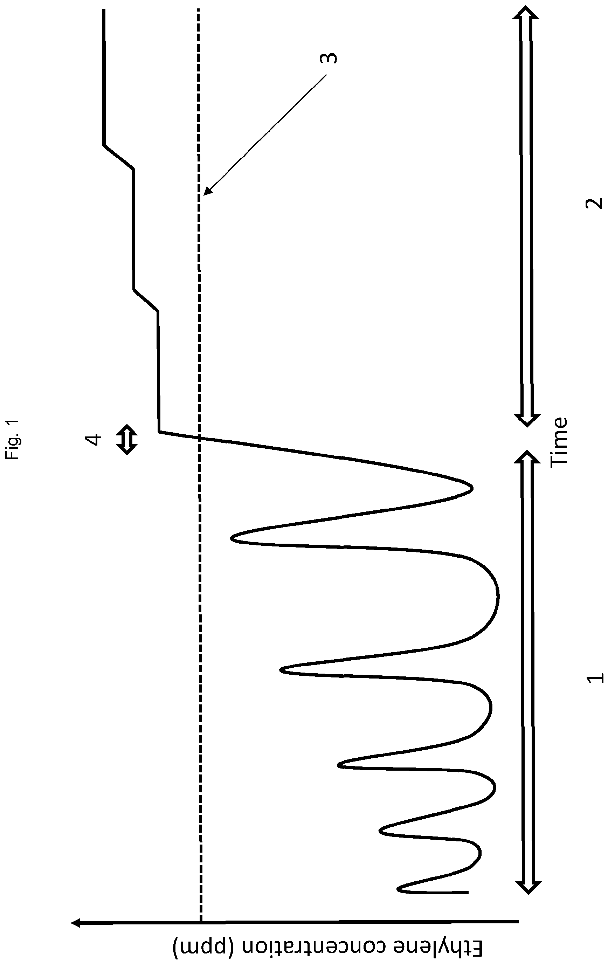

An embodiment of the present invention is described, by way of example only, with reference to Figure 1.

Figure 1 is a graph illustrating phases of an ethylene environment control method according to an embodiment.

In a pump control phase (1), a super low dose (between 0.001-0.099ppm) of ethylene is provided a crop store for a short period between 1-10hr every 24 hours. Such concentrations cannot be measured by instruments except those in laboratories and so the ethylene control at this stage is by metering by the ethylene generating or dispensing system. The number and (optionally) a peak output of ethylene phases can be selected. This is called the Pump Control phase. During this phase, each subsequent dose is increased by a predetermined increment.

As the store ethylene concentration increases during the Pump Control phase (1) it eventually reaches a concentration (3) that is detected by an ethylene sensor in the crop store. After a qualifying period (4), where detected ethylene concentration is greater than a pre-determined value and for a pre-determined period of time, control transfers to the sensor, under a sensor control phase (2). In the Sensor control phase (2), a reduction in store ethylene concentration below a pre-determined value is detected by the sensor and this triggers the introduction of additional ethylene in order to maintain the pre-determined concentration.

In the example shown in Figure 1, in the Pump control phase (1), ethylene is generated in six periods, each to an increasing concentration. Ethylene concentrations in this phase are very low, typically less than 0.1ppm. During the sixth period, a pre-determined ethylene concentration (3) is achieved for a pre-determined time. This is the Qualifying period (4) and prompts a change from Pump control phase (1) to the Sensor control phase (2). Ethylene concentration is managed by direct measurement and feedback control loop or similar in the Sensor control phase.

Preferably, the number and peak output of periods during the Pump Control phase is configurable. This may, for example, be to accommodate different crop store sizes. If the pump were to dispense 5ml of ethanol (for making ethylene) this would have a much bigger impact in a 500 tonne capacity store than in a 5,000 tonne store.

It is to be appreciated that certain embodiments of the invention as discussed below may be incorporated as code (e.g., a software algorithm or program) residing in firmware and/or on computer useable medium having control logic for enabling execution on a computer system having a computer processor. Such a computer system typically includes memory storage configured to provide output from execution of the code which configures a processor in accordance with the execution. The code can be arranged as firmware or software, and can be organized as a set of modules such as discrete code modules, function calls, procedure calls or objects in an object-oriented programming environment. If implemented using modules, the code can comprise a single module or a plurality of modules that operate in cooperation with one another.

Optional embodiments of the invention can be understood as including the parts, elements and features referred to or indicated herein, individually or collectively, in any or all combinations of two or more of the parts, elements or features, and wherein specific integers are mentioned herein which have known equivalents in the art to which the invention relates, such known equivalents are deemed to be incorporated herein as if individually set forth.

Although illustrated embodiments of the present invention have been described, it should be understood that various changes, substitutions, and alterations can be made by one of ordinary skill in the art without departing from the present invention which is defined by the recitations in the claims below and equivalents thereof.

## Claims

1. A method for providing an ethylene environment in a crop store comprising:
monitoring ethylene concentration in the crop store
setting an ethylene acclimatisation dosage to an initial value;
performing pump control including repeating:
applying an ethylene dosage to the crop store according to the acclimatisation dosage;
waiting for a predetermined period; and,
increasing the acclimatisation dosage value for a next dosage;
Upon the monitoring detecting an ethylene concentration for a qualification period, changing from pump control to sensor control, sensor control comprising:
monitoring ethylene concentration in the crop store; and,
upon the ethylene concentration falling below a predetermined threshold, triggering addition of ethylene to the crop store.

2. The method of claim 1, wherein the initial value of the ethylene acclimatisation dosage is 0.001-0.099ppm.

3. The method of claim 1 or 2, wherein the predetermined period is selected in dependence on the crop store whereby ethylene concentration is substantially 0 by the end of period.

4. The method of claim 1, 2 or 3, wherein the pump control includes metering dosage from an ethylene generating or dispensing system that doses ethylene according to the acclimatisation dosage.

5. The method of any preceding claim, wherein the sensor control includes applying a ramped or stepped concentration of ethylene over time.

6. The method of any preceding claim, wherein the pump control operates in dependence on one or more parameters selected from a set including number of repetitions to be performed and peak output of ethylene into crop store.

7. An ethylene environment control system comprising:
a crop store;
an ethylene sensor configured to monitor ethylene concentration in the crop store;
an ethylene source having an ethylene dosage set to an acclimatisation value, the acclimatisation value being below the concentration detectable by the ethylene sensor;
a controller, wherein the controller is configured to:
monitor the ethylene sensor for detection of ethylene;
performing pump control including repeating:
causing the ethylene source to apply an ethylene dosage to the crop store according to the acclimatisation dosage;
waiting for a predetermined period; and,
increasing the acclimatisation dosage value for a next dosage;
upon the ethylene sensor detecting an ethylene concentration for a qualification period, changing from pump control to sensor control, sensor control comprising:
monitoring ethylene concentration in the crop store using the ethylene sensor; and,
upon the ethylene concentration falling below a predetermined threshold, triggering addition of ethylene to the crop store by the ethylene source.
